# EUROPEAN PATENT SPECIFICATION

(11) **EP 3 247 522 B1**
(45) Date of publication and mention of the grant of the patent: **13.09.2023**
(21) Application number: 15820714.2
(22) Date of filing: 11.12.2015
(51) Int. Cl.: B23K 9/095, A61F 9/06, B23K 9/32, G09B 19/24

(54) **USER CONFIGURATION OF IMAGE CAPTURE AND DISPLAY IN A WELDING VISION SYSTEM**
BENUTZERKONFIGURATION EINER BILDERFASSUNG UND -ANZEIGE IN EINEM SCHWEISSSICHTSYSTEM
CONFIGURATION UTILISATEUR DE CAPTURE ET D'AFFICHAGE D'IMAGES DANS UN SYSTÈME DE VISION ARTIFICIELLE DE SOUDAGE

(30) Priority: 23.01.2015 US 201514604210
(43) Date of publication of application: 29.11.2017
(73) Proprietor: Illinois Tool Works Inc., Glenview, IL 60025 (US)
(72) Inventor: BEESON, Richard, Glenview, Illinois 60025 (US); BECKER, William J., Glenview, Illinois 60025 (US)
(74) Representative: Meissner Bolte Partnerschaft mbB
(86) International application number: PCT/US2015/065209
(87) International publication number: WO 2016/118247

(56) References cited:
- US-A1- 2009 231 423
- US-A1- 2012 291 172

## Description

### BACKGROUND

The invention relates generally to welding systems, and more particularly, to a system for recording welding operations for later review, analysis, teaching, and so forth as defined in the preamble of claim 1. US 2009/0231 423 A1 (which forms the basis for the preamble of the independent claim) discloses a weld recording system mounted in or on a welding helmet. The weld recording system includes a camera assembly unit, a power supply unit, a processor, and removable memory. The weld recording system interfaces with lens control circuitry, an optical sensor, a welding power supply, and a helmet position sensor. Logic is provided for the triggering and recording of video and audio signals, which are eventually stored in a file for future reference. Transmission of signals from one or more welders to a monitoring station for eventual display is presented. An image processing algorithm is provided to combine multiple images with varied exposure times into a visual image of the weld and its surroundings.

US 2012/0291 172 A1 also shows a similar welding system.

Welding is a process that has increasingly become ubiquitous in all industries. While such processes may be automated in certain contexts, a large number of applications continue to exist for manual welding operations performed by skilled welding technicians. However, as the average age of the skilled welder rises, the future pool of qualified welders is diminishing. Furthermore, many inefficiencies plague the welding training process, potentially resulting in injecting a number of improperly trained students into the workforce, while discouraging other possible young welders from continuing their education. For instance, class demonstrations do not allow all students clear views of the welding process. Additionally, instructor feedback during student welds is often prohibited by environmental constraints.
An object of the present invention is to enable also an unexperienced user to select the best image display and/or capture settings for a welding process. This object is achieved by the featues of claim 1.

### BRIEF SUMMARY OF THE INVENTION

A video presentation of a welding operation is presented to a helmet of a welder of the welding operation. A welding operation on a sample workpiece is captured on video and stored. The stored captured video is played back to the welder on the display within the welder's helmet. The welder views the play back while adjusting the play characteristics of the camera. After the adjustment, a second live welding operation is conducted and displayed to the welder using the adjusted characteristics.

### BRIEF DESCRIPTION OF THE DRAWINGS

FIG. 1 shows an arc welding system in accordance with the present invention.
FIG. 2 is a block diagram of welding equipment of the system of FIG. 1.
FIG. 3 is a perspective front side view of welding headwear of the system of FIG. 1.
FIG. 4 is a block diagram of circuitry of the headwear of FIG. 3.
FIGS. 5A-5C illustrate various parameters which may be determined from images of a weld in progress.
FIG. 6 is a flowchart illustrating an example process for configuring and operating the welding headwear of FIGS. 3 and 4.
FIG. 7 shows side-by-side viewing of different image capture settings and/or image display settings.

### DETAILED DESCRIPTION OF THE INVENTION

Aspects of the present disclosure provide a methods and systems for capturing and reviewing welding operations. The methods and systems allows for capturing video and audio data during a welding operation, along with, where desired, actual welding parameters measured or calculated at times corresponding to the video and audio data. In an example implementation of this disclosure, a weld recording system is mounted in or on a welding helmet that includes a camera assembly unit, a power supply unit, a processor, and removable memory. The weld recording system may interface with lens control circuitry, an optical sensor, a welding power supply, and/or a helmet position sensor. Logic may be provided for the triggering and recording of video and audio signals, which may be stored in a file for future reference.

Signals may be transmitted from one or more such weld recording systems to a monitoring station for display. In an example implementation of this disclosure, an image processing algorithm is performed to combine multiple images with varied parameters (e.g., exposure times, aperature settings, and/or the like) into a visual image of the weld and its surroundings. In an example implementation, real-time playback is provided, such as for instruction, monitoring, and so forth.

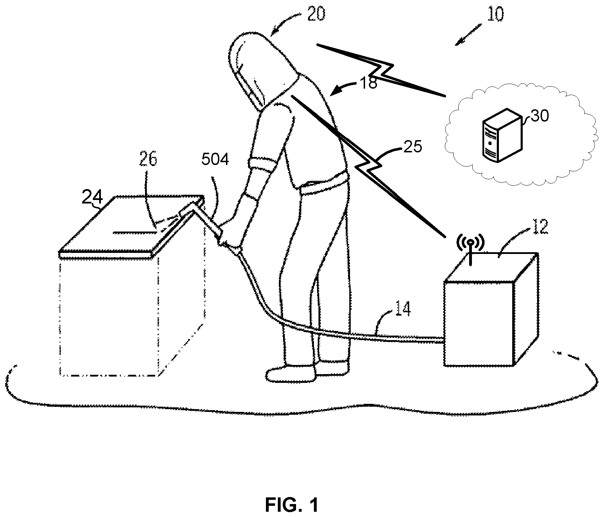

Referring to FIG. 1, there is shown an example welding system 10 in which a welder/operator 18 is wearing welding headwear 20 and welding a workpiece 24 using a torch 504 to which power or fuel is delivered by equipment 12 via a conduit 14. The equipment 12 may comprise a power or fuel source, optionally a source of an inert shield gas and, where wire/filler material is to be provided automatically, a wire feeder. The welding system 10 of FIG. 1 may be configured to form a weld joint 512 by any known technique, including flame welding techniques such as oxy-fuel welding and electric welding techniques such shielded metal arc welding (i.e., stick welding), metal inert gas welding (MIG), flux cored arc welding (FCAW) tungsten inert gas welding (TIG), and resistance welding. TIG welding may involve no external filler metal or may involve manual, automated or semi-automated external metal filler.

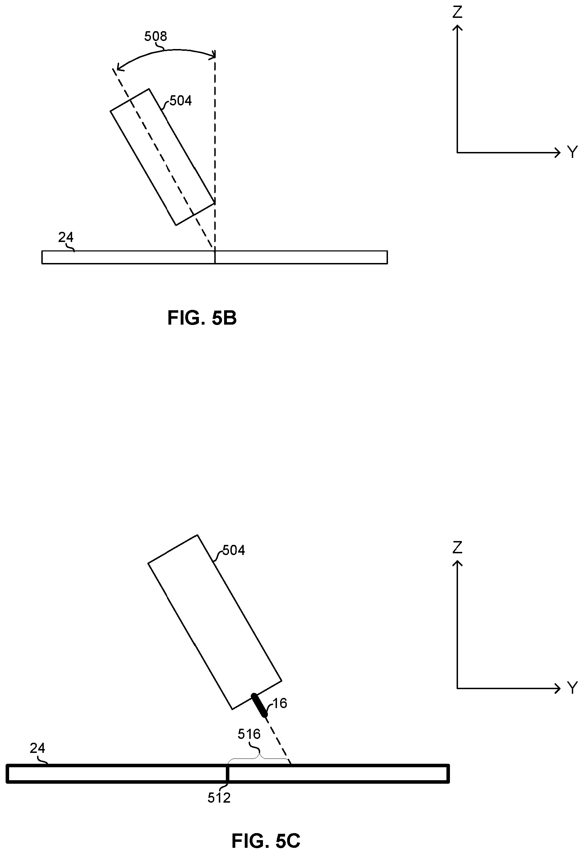

Optionally in any embodiment, the welding equipment 12 may be arc welding equipment that provides a direct current (DC) or alternating current (AC) to a consumable or non-consumable electrode 16 (better shown, for example, in FIG. 5C) of a torch 504, which may be a TIG torch, a MIG or flux cored torch (commonly called a MIG "gun"), or a stick electrode holder (commonly called a "stinger"). The electrode 16 delivers the current to the point of welding on the workpiece 24. In the welding system 10, the operator 18 controls the location and operation of the electrode 16 by manipulating the torch 504 and triggering the starting and stopping of the current flow. When current is flowing, an arc 26 is developed between the electrode and the workpiece 24. The conduit 14 and the electrode 16 thus deliver current and voltage sufficient to create the electric arc 26 between the electrode 16 and the workpiece. The arc 26 locally melts the workpiece 24 and welding wire or rod supplied to the weld joint 512 (the electrode 16 in the case of a consumable electrode or a separate wire or rod in the case of a non-consumable electrode) at the point of welding between electrode 16 and the workpiece 24, thereby forming a weld joint 512 when the metal cools.

As shown, and described more fully below, the equipment 12 and headwear 20 may communicate via a link 25 via which the headwear 20 may control settings of the equipment 12 and/or the equipment 12 may provide information about its settings to the headwear 20. Although a wireless link is shown, the link may be wireless, wired, or optical.

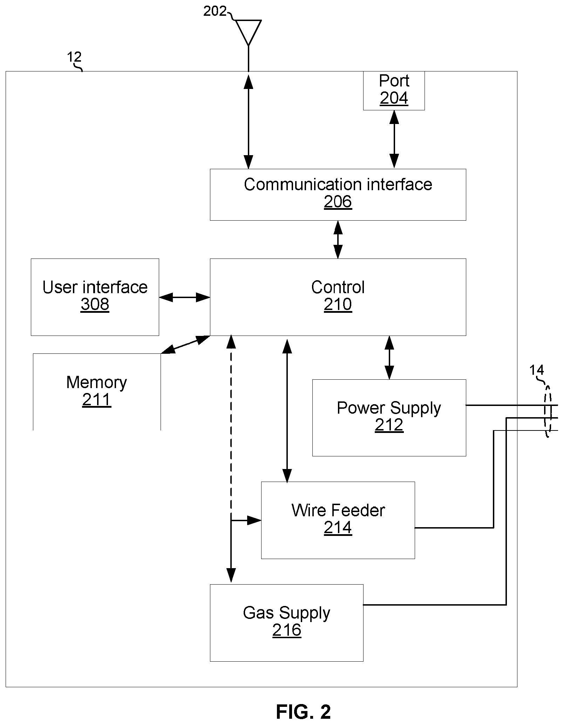

FIG. 2 shows example welding equipment in accordance with aspects of this disclosure. The equipment 12 of FIG. 2 comprises an antenna 202, a communication port 204, communication interface circuitry 206, user interface module 208, control circuitry 210, power supply circuitry 212, wire feeder module 214, and gas supply module 216.

The antenna 202 may be any type of antenna suited for the frequencies, power levels, etc. used by the communication link 25.

The communication port 204 may comprise, for example, an Ethernet over twisted pair port, a USB port, an HDMI port, a passive optical network (PON) port, and/or any other suitable port for interfacing with a wired or optical cable.

The communication interface circuitry 206 is operable to interface the control circuitry 210 to the antenna 202 and/or port 204 for transmit and receive operations. For transmit operations, the communication interface 206 may receive data from the control circuitry 210 and packetize the data and convert the data to physical layer signals in accordance with protocols in use on the communication link 25. For receive operations, the communication interface may receive physical layer signals via the antenna 202 or port 204, recover data from the received physical layer signals (demodulate, decode, etc.), and provide the data to control circuitry 210.

The user interface module 208 may comprise electromechanical interface components (e.g., screen, speakers, microphone, buttons, touchscreen, etc.) and associated drive circuitry. The user interface 208 may generate electrical signals in response to user input (e.g., screen touches, button presses, voice commands, etc.). Driver circuitry of the user interface module 208 may condition (e.g., amplify, digitize, etc.) the signals and them to the control circuitry 210. The user interface 208 may generate audible, visual, and/or tactile output (e.g., via speakers, a display, and/or motors/actuators/servos/etc.) in response to signals from the control circuitry 210.

The control circuitry 210 comprises circuitry (e.g., a microcontroller and memory) operable to process data from the communication interface 206, the user interface 208, the power supply 212, the wire feeder 214, and/or the gas supply 216; and to output data and/or control signals to the communication interface 206, the user interface 208, the power supply 212, the wire feeder 214, and/or the gas supply 216.

The power supply circuitry 212 comprises circuitry for generating power to be delivered to a welding electrode via conduit 14. The power supply circuitry 212 may comprise, for example, one or more voltage regulators, current regulators, inverters, and/or the like. The voltage and/or current output by the power supply circuitry 212 may be controlled by a control signal from the control circuitry 210. The power supply circuitry 212 may also comprise circuitry for reporting the present current and/or voltage to the control circuitry 210. In an example implementation, the power supply circuitry 212 may comprise circuitry for measuring the voltage and/or current on the conduit 14 (at either or both ends of the conduit 14) such that reported voltage and/or current is actual and not simply an expected value based on calibration.

The wire feeder module 214 is configured to deliver a consumable wire electrode 16 to the weld joint 512 (FIG. 5C). The wire feeder 214 may comprise, for example, a spool for holding the wire, an actuator for pulling wire off the spool to deliver to the weld joint 512, and circuitry for controlling the rate at which the actuator delivers the wire. The actuator may be controlled based on a control signal from the control circuitry 210. The wire feeder module 214 may also comprise circuitry for reporting the present wire speed and/or amount of wire remaining to the control circuitry 210. In an example implementation, the wire feeder module 214 may comprise circuitry and/or mechanical components for measuring the wire speed, such that reported speed is actual and not simply an expected value based on calibration.

The gas supply module 216 is configured to provide shielding gas via conduit 14 for use during the welding process. The gas supply module 216 may comprise an electrically controlled valve for controlling the rate of gas flow. The valve may be controlled by a control signal from control circuitry 210 (which may be routed through the wire feeder 214 or come directly from the control 210 as indicated by the dashed line). The gas supply module 216 may also comprise circuitry for reporting the present gas flow rate to the control circuitry 210. In an example implementation, the gas supply module 216 may comprise circuitry and/or mechanical components for measuring the gas flow rate such that reported flow rate is actual and not simply an expected value based on calibration.

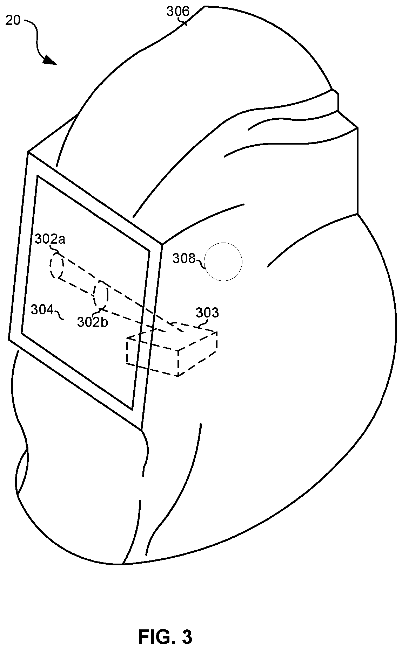

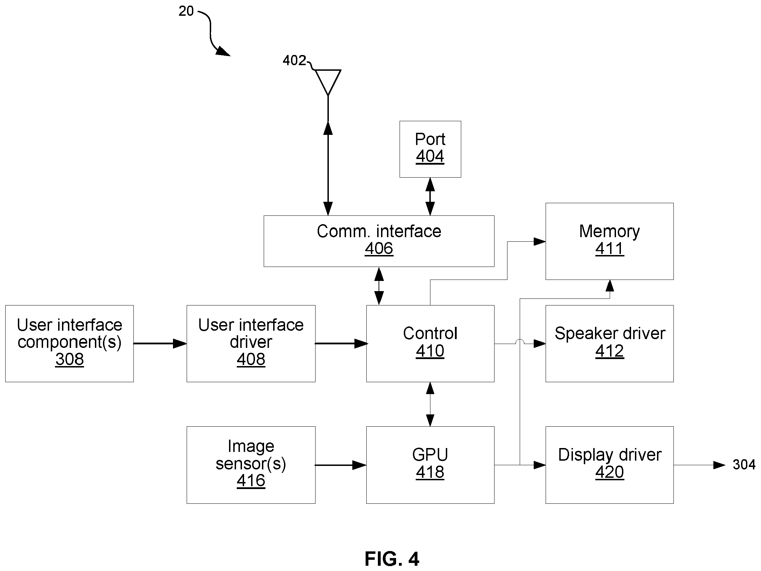

Referring to FIGS. 3 and 4, helmet 20 comprises a shell 306 in or to which are mounted: one or more cameras 303, a display 304, electromechanical user interface 308, an antenna 402, a communication port 404, a communication interface 406, a user interface driver 408, a central processing unit (CPU) control circuitry 410, speaker driver circuitry 412, a graphics processing unit (GPU) 418, and display driver circuitry 420. Each of the cameras 303 comprises one or more optical components 302 and image sensor(s) 416. In other embodiments, helmet 20 may take the form of a mask or goggles, for example.

Each of the camera's optical components 302a, 302b comprises, for example, one or more lenses, filters, and/or other optical components for capturing electromagnetic waves in the spectrum ranging from, for example, infrared to ultraviolet. Optical components 302a, 302b are for two cameras respectively and are positioned approximately centered with the eyes of a wearer of helmet 20 to capture stereoscopic images (at any suitable frame rate ranging from still photos to video at 30 fps, 100 fps, or higher) of the field of view the wearer of helmet 20 as if looking through a lens.

Display 304 may comprise, for example, a LCD, LED, OLED. E-ink, and/or any other suitable type of display operable to convert electrical signals into optical signals viewable by a wearer of helmet 20.

The electromechanical user interface components 308 may comprise, for example, one or more touchscreen elements, speakers, microphones, physical buttons, etc. that generate electric signals in response to user input. For example, electromechanical user interface components 308 may comprise capacity, inductive, or resistive touchscreen sensors mounted on the back of the display 304 (i.e., on the outside of the helmet 20) that enable a wearer of the helmet 20 to interact with user interface elements displayed on the front of the display 304 (i.e., on the inside of the helmet 20). In an example implementation, the optics 302, image sensors 416, and GPU 418 may operate as user interface components 308 by allowing a user to interact with the helmet 20 through, for example, hand gestures captured by the optics 302 and images sensors 416 and then interpreted by the GPU 418. For example, a gesture such as would be made to turn a knob clockwise may be interpreted to generate a first signal while a gesture such as would be made to turn a knob counterclockwise may be interpreted to generate a second signal.

Antenna 402 may be any type of antenna suited for the frequencies, power levels, etc. used by communication link 25.

Communication port 404 may comprise, for example, an Ethernet over twisted pair port, a USB port, an HDMI port, a passive optical network (PON) port, and/or any other suitable port for interfacing with a wired or optical cable.

Communication interface circuitry 406 is operable to interface control circuitry 410 to the antenna 402 and port 404 for transmit and receive operations. For transmit operations, communication interface 406 receives data from control circuitry 410, and packetizes the data and converts the data to physical layer signals in accordance with protocols in use by communication link 25. The data to be transmitted may comprise, for example, control signals for controlling the equipment 12. For receive operations, communication interface 406 receives physical layer signals via antenna 402 or port 404, recovers data from the received physical layer signals (demodulate, decode, etc.), and provides the data to control circuitry 410. The received data may comprise, for example, indications of current settings and/or actual measured output of equipment 12 (e.g., voltage, amperage, and/or wire speed settings and/or measurements).

User interface driver circuitry 408 is operable to condition (e.g., amplify, digitize, etc.) signals from user interface components 308.

Control circuitry 410 is operable to process data from communication interface 406, user interface driver 408, and GPU 418, and to generate control and/or data signals to be output to speaker driver circuitry 412, GPU 418, and communication interface 406.

Signals output to communication interface 406 may comprise, for example, signals to control the settings of equipment 12. Such signals may be generated based on signals from GPU 418 and/or the user interface driver 408.

Signals from communication interface 406 comprise, for example, indications (received via antenna 402, for example) of current settings and/or actual measured output of equipment 12.

Speaker driver circuitry 412 is operable to condition (e.g., convert to analog, amplify, etc.) signals from control circuitry 410 for output to one or more speakers of user interface components 308. Such signals may, for example, carry audio to alert a wearer of helmet 20 that a welding parameter is out of tolerance, to provide audio instructions to the wearer of helmet 20, etc. For example, if the travel speed of the torch is determined to be too slow, such an alert may comprise a voice saying "too slow."

Signals to GPU 418 comprise, for example, signals to control graphical elements of a user interface presented on display 304. Signals from the GPU 418 comprise, for example, information determined based on analysis of pixel data captured by images sensors 416. Image sensor(s) 416 may comprise, for example, CMOS or CCD image sensors operable to convert optical signals from cameras 303 to digital pixel data and output the pixel data to GPU 418.

Graphics processing unit (GPU) 418 is operable to receive and process pixel data (e.g., of stereoscopic or two-dimensional images) from image sensor(s) 416. GPU 418 outputs one or more signals to the control circuitry 410, and outputs pixel data to the display 304 via display driver 420.

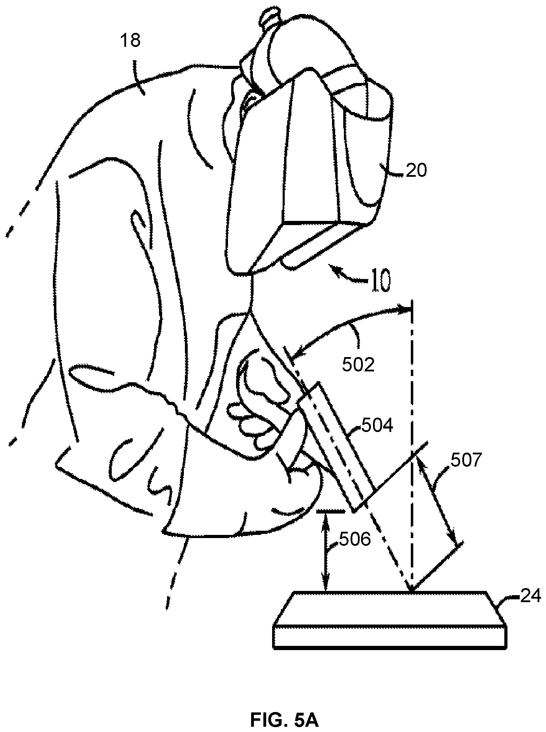

The processing of pixel data by GPU 418 may comprise, for example, analyzing the pixel data, e.g., a barcode, part number, time stamp, work order, etc., to determine, in real time (e.g., with latency less than 100ms or, more preferably, less than 20ms, or more preferably still, less than 5ms), one or more of the following: name, size, part number, type of metal, or other characteristics of workpiece 24; name, size, part number, type of metal, or other characteristics of torch 504, electrode 16 and/or filler material; type or geometry of joint 512 to be welded; 2-D or 3-D positions of items (e.g., electrode, workpiece, etc.) in the captured field of view, one or more weld parameters (e.g., such as those described below with reference to FIGS. 5A, 5B and 5C) for an in-progress weld in the field of view; measurements of one or more items in the field of view (e.g., size of a joint or workpiece being welded, size of a bead formed during the weld, size of a weld puddle formed during the weld, and/or the like); and/or any other information which may be gleaned from the pixel data and which may be helpful in achieving a better weld, training the operator, calibrating the system 10, etc.

The information output from GPU 418 to control circuitry 410 may comprise the information determined from the pixel analysis.

The pixel data output from GPU 418 to display 304 may provide a mediated reality view for the wearer of helmet 20. In such a view, the wearer experiences a video presented on display 304 as if s/he is looking through a lens. The image may be enhanced and/or supplemented by an on-screen display. The enhancements (e.g., adjust contrast, brightness, saturation, sharpness, etc.) may enable the wearer of helmet 20 to see things s/he could not see with simply a lens. The on-screen display may comprise text, graphics, etc. overlaid on the video to provide visualizations of equipment settings received from control circuit 410 and/or visualizations of information determined from the analysis of the pixel data.

Display driver circuitry 420 is operable to generate control signals (e.g., bias and timing signals) for display 304 and to condition (e.g., level control synchronize, packetize, format, etc.) pixel data from GPU 418 for conveyance to display 304.

FIGS. 5A-5C illustrate various parameters which may be determined from images of a weld in progress. Coordinate axes are shown for reference. In FIG. 5A, the Z axis points to the top of the paper, the X axis points to the right, and the Y axis points into the paper. In FIGS. 5B and 5C, the Z axis points to the top of the paper, the Y axis points to the right, and the X axis points into the paper.

In FIGS. 5A-5C, equipment 12 comprises a MIG gun 504 that feeds a consumable electrode 16 to a weld joint 512 of workpiece 24. During the welding operation, a position of the MIG gun 504 may be defined by parameters including: contact-tip-to-work distance 506 or 507, a travel angle 502, a work angle 508, a travel speed 510, and aim.

Contact-tip-to-work distance may include a vertical distance 506 from a tip of torch 504 to workpiece 24 as illustrated in FIG. 5A. In other embodiments, the contact-tip-to-work distance may be a distance 507 from the tip of torch 504 to workpiece 24 at the angle of torch 504 to workpiece 24.

The travel angle 502 is the angle of gun 504 and/or electrode 16 along the axis of travel (X axis in the example shown in FIGS. 5A-5C).

A work angle 508 is the angle of gun 504 and/or electrode 16 perpendicular to the axis of travel (Y axis in the example shown in FIGS. 5A-5C).

The travel speed is the speed at which gun 504 and/or electrode 16 moves along the joint 512 being welded.

The aim is a measure of the position of electrode 16 with respect to the joint 512 to be welded. Aim may be measured, for example, as distance from the center of the joint 512 in a direction perpendicular to the direction of travel. FIG. 5C, for example, depicts an example aim measurement 516.

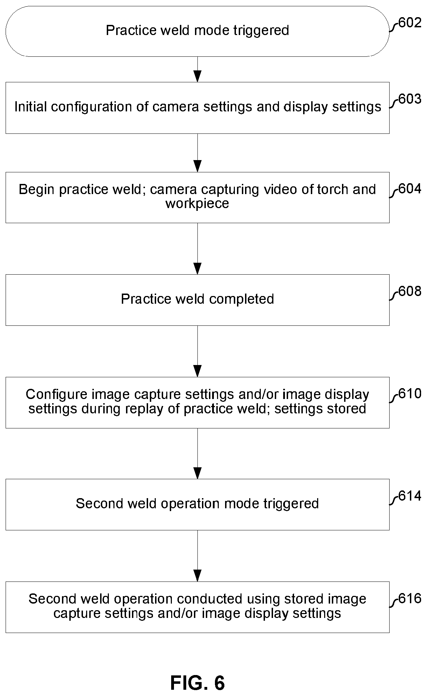

Referring to FIG. 6, the flowchart illustrates a process for a welding a workpiece 24. An initial workpiece to be welded is a sample workpiece, often called a "coupon." That is, the initial workpiece may, for example, be a scrap piece of metal having characteristics (e.g., type of metal, size of joint to be welded, and/or the like.) that are the same as or similar to a second workpiece to be later welded in a second weld operation.

In block 602, welder 18 sets up for a practice weld. The sample workpiece is placed into position, together with the electrode, relative to the field of view of camera lenses 302a, 302b. Also, one or more settings of equipment 12 is configured by the welder 18 using user interface components 308. For example, signals from the helmet 20 to equipment 12 may select a constant current or constant voltage mode, set a nominal voltage and/or nominal current, set a voltage limit and/or current limit, set a wire speed, and/or the like. Welder 18 then initiates a live practice weld mode. For example, welder 18 may give a voice command to enter the live practice weld mode which command is responded to by user interface components 308 of helmet 20. Control circuitry 410 configures the components of helmet 20 according to the command in order to display on display 304 the first live practice weld for viewing by the welder. The welder views the weld on display 304 and controls operation and positioning of electrode 16. Control circuitry 410 may also respond to the voice command and send a signal to equipment 12 to trigger the practice weld mode in equipment 12. For example, control circuitry 210 disables a lock out so that power is delivered to electrode 16 via power supply 212 when a trigger on the torch is pulled by the welder. Wire feeder 214 and gas supply 216 may also be activated accordingly. Block 602 thus represents the step of the welder placing the welding system in a weld mode so that the sample workpiece may be welded.

In block 603, initial image capture settings and/or image display settings are configured. Image capture settings may comprise, for example, settings of optics 302 (e.g., aperture, focal length, filter darkness, etc.) and settings of image sensor(s) 416 (e.g., exposure times, bias currents and/or voltages, and/or the like.) Image display settings may comprise, for example, general image processing settings such as brightness, contrast, sharpness, color, hue, and/or the like of images processed by the GPU 418 and display driver 420 and displayed on display 304. Image display settings may be set in the GPU 418, the display driver 420, and/or display 304.

Image display settings may also (or alternatively) comprise, for example, settings of parameters that control the combining of pixel data from two or more image sensors 416. In an example implementation, a first image sensor having a darker filter ("dark" image sensor) and a second image sensor having a lighter filter ("light" image sensor" may capture the same field of view and GPU 418 may implement an algorithm to decide how to combine the pixel data from the two sensors. For example, for each pixel, the algorithm may determine whether to use entirely the pixel data from the dark image sensor, entirely the pixel data from the light image sensor, or a weighted combination of pixel data from both of the sensors.

Image display settings may also (or alternatively) comprise welding-specific image processing settings such as "puddle enhancement" and "joint enhancement" settings, which determine, for example, how pixels from multiple image sensors are combined and/or how general image processing settings are applied on a pixel-by-pixel (or group-of-pixels by group-of-pixel) basis.

Still referring to block 603, in an example implementation, the initial image capture settings and/or initial image display settings may be manually selected by welder wearing the helmet 20 via the user interface components 308), automatically selected by circuitry of the welding helmet 20, or a combination of the two (e.g., the circuitry provides multiple options for the image capture settings and/or image display settings and the welder selects from among the options. In the automatic, or semiautomatic case, the circuitry of the helmet may select (or recommend) initial image capture and/or initial image display settings based on characteristics of the weld to be performed. The characteristics of the weld to be performed may be determined from known information about the weld (e.g., from an electronic work order retrieved from server 30). Alternatively (or additionally), the characteristics of the weld to be performed may be determined from image analysis performed by the helmet 20. Characteristics of the weld to be performed may include, for example: the type of metal to be welded, the type of torch to be used, the type of filler material to be used, the size and/or angle of the joint to be welded, the wire speed settings to be used, the voltage and/or amperage to be used, the ambient conditions (humidity, lighting, temperature, and/or the like.) in which the weld is to be performed, target/nominal welding parameters to be used for the weld, actual welding parameters determined in real-time from analysis of the captured images, and/or the like. The characteristics may be used, for example, to predict arc brightness and the initial image capture settings and/or initial image display settings may be configured to accommodate such arc brightness. The characteristics may be used, for example, to predict image contrast and the initial image capture settings and/or initial image display settings may be configured to accommodate such contrast.

In block 604, welder 18 activates the trigger of the torch 504, and images of the weld operation begin to be captured by the camera 303 and presented onto display 304. The pixel data of the image is also stored. The pixel data may be stored in a multimedia file located in a separate storage 30, and/or the pixel data may be stored in a multimedia file located in a memory 411 located in helmet 20, and/or in a memory 211 located in equipment 12. For example, when the operator pulls the trigger, camera(s) 303 begin capturing images (e.g., at 30 frames per second or higher). The operator begins welding, moving electrode 16 relative to the joint to be welded with power being delivered to electrode 16. The electrode 16 proceeds along the joint during welding, and the captured pixel data is processed and stored. In an example implementation, these events may be sequenced such that image capture starts first and allows a few frames during which the aforementioned block 603 takes place. This may ensure sufficient image quality even at the very beginning of the welding operation.

In an example implementation, raw data from the image sensor(s) may be stored. In an example implementation, pixel data may be stored after being processed by the GPU 418. In an example implementation, image capture settings may be varied during the practice weld such that different frames of the stored video are representative of different image capture settings.

In block 608, the first weld operation on the sample workpiece is completed
In block 610, the welder replays the stored video while wearing helmet 20, playing the video back onto the display 304 of helmet 20. User interface components 308 are manipulated by the welder to cause replay of the weld video. Control circuitry 410 receives the request signal for replay via user interface driver 408. Control circuitry 410 then retrieves the stored video data from memory, e.g., from memory 30 via antenna 402 or port 404, and provides the retrieved data to GPU 418 which processes the video data and outputs it to display 304 via display driver 420.

During replay, the welder 18 can focus full attention onto the presentation of the video to display 304, since attention need not be given to manual manipulation of the electrode 16. Since the video is representative of what the welder 18 will be seeing when he performs the second weld, the video provides a good point of reference for selecting image capture settings and/or image display settings to be used during a subsequent weld. The welder 18 may, using user interface components 308, cycle between frames representing various image capture settings and, based on which of those frames looks best to him/her, select those as the image capture settings to be used for a subsequent weld. Similarly, the welder may, using user interface components 308, adjust image display settings and, based on which of image display settings look best to him/her and/or provide a desired effect (e.g., puddle enhancement, joint enhancement, etc.), select those as the image display settings to be used for a subsequent weld. Once the welder 18 arrives at image capture settings and/or image display settings that s/he finds to provide an optimal view of the welding process, the welder 18 may trigger a save of such settings to memory. (e.g., to memory 211, 411, and/or memory of server 30). The settings may be associated in memory with a user profile of the welder 18, such that the welder 18 can recall them at a later time, and even on a different helmet 20.

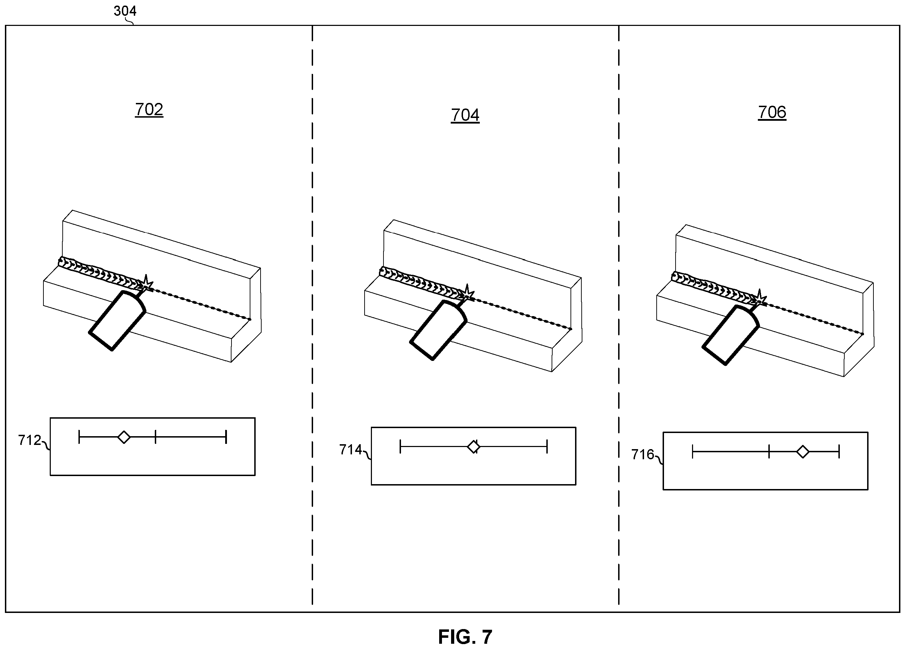

In an example implementation, video frames may be scaled and/or cropped during replay such that multiple frames of the recording can be presented simultaneously on the display 304 for side-by-side comparison of different image capture and/or different image display settings. An example of such an implementation is described below with reference to FIG. 7.

In block 614, the welder places the welding system in a weld mode in order to perform a second live welding operation on a second workpiece 24. This is performed similar to block 602, discussed above. This second workpiece is not a sample, but rather the intended original workpiece to be welded.

In block 616, the second live weld operation is conducted. During the second live welding operation, the image capture settings and/or image display settings stored in block 612 are utilized for capturing and/or displaying real-time images of the second weld operation.

In an example implementation, video frames may be scaled and/or cropped during real-time playback such that multiple versions of the real-time images can be presented simultaneously on the display 304 for side-by-side viewing of different image capture settings and/or different image display settings. For example, alternate frames may be captured with different image capture settings and/or image display settings and presented in real-time side-by-side on the display 304. One of the frames may have image capture and/or image display settings that improve viewing of a first portion of feature of the images (e.g., the arc) and the other of the frames may have image capture and/or image display settings that improve viewing of a second portion or feature of the images (e.g., the seam). An example of such an implementation is described below with reference to FIG. 7. Alternatively, the alternate frames may not both be shown simultaneously but the welder 18 may readily switch between (e.g., using voice commands), for example, odd frames captured and displayed with first settings and even frames captured and displayed with second settings.

Now referring to FIG. 7, shown is an example implementation in which multiple images are presented side-by-side on the display 304 of the helmet 20. Shown are three images 702, 704, and 706.

In some instances, each of the images 702, 704, 706 may be different frames of the stored video of the practice weld. In such an instance, each of the frames may have been captured using different image capture settings. Accordingly, the three images 702, 704, 706 provide for side-by-side comparison of the different image capture settings such that the welder 18 can determine which image capture settings s/he thinks result in optimal viewing of the video. Since the practice weld in the video shares most, if not all, characteristics with a subsequent weld to be performed, use of such settings for a the subsequent weld will likely provide the welder 18 with a good view of the subsequent weld. In such an instance, the graphical overlays 712, 714, and 716 may show the image capture settings that were used for their respective images.

In some instances, each of the images 702, 704, 706 may be the same frame of the stored video of the practice weld, but with different image display settings applied. Accordingly, the three images 702, 704, 706 provide for side-by-side comparison of the different image display settings such that the welder 18 can determine which image display settings s/he thinks result in optimal viewing of the video. Since the practice weld in the video shares most, if not all, characteristics with a subsequent weld to be performed, use of such settings for a the subsequent weld will likely provide the welder 18 with a good view of the subsequent weld. In such an instance, the graphical overlays 712, 714, and 716 may show the image display settings being applied to for their respective images.

According to the invention, welding headwear (e.g., helmet 20) comprises a camera (e.g., 303), user interface components (308), a display (e.g., 304), memory (e.g., 411), and processing circuitry (e.g., 308, 408, 410, 418 and 420). The welding headwear is operable to: capture, via the camera, images of a first live welding operation performed on a sample workpiece; store, to the memory, the captured images of the first live welding operation; play back, on the display, the stored images of the first live welding operation; select, during the play back and based on the captured images, image capture settings of the welding headwear to be used for a second live welding operation; capture, via the camera, images of a second live welding operation using the selected image capture settings; and display, on the display in real-time, the images of the second live welding operation. The welding headwear is further operable to select (with or without user input), during the play back and based on the captured images, image display settings of the welding headwear to be used for the second live welding operation. The welding headwear is also operable to apply the selected images display settings to the images of the second live weld operation during the display of the images of the second live weld operation. The image capture settings comprise settings of optical components (e.g., 302) of the camera. The settings of the optical components may comprise one or more of: focal length, aperture, and exposure time. The image capture settings may comprise settings of an image sensor (e.g., 416) of the camera. The settings of the image sensor may comprise one or more of: exposure time, bias voltage, and bias current. The welding headwear may be operable to configure the camera to use, during the capture of the images of the first live weld operation, different image capture settings for different ones of the captured images of the first live welding operation. The welding headwear may be operable to display, on the display, different ones of the captured images side-by-side during the play back. The welding headwear may be operable to display, on the display, multiple versions of one of the captured images of the first live welding operation. Each of the multiple versions of the one of the captured images of the first live welding operation may be displayed with different image display settings. The welding headwear may be operable to perform the selection automatically based on weld characteristics. The welding headwear may be operable to determine the weld characteristics based on processing of the captured images of the first live welding operation. The welding headwear may be operable to: capture, via the camera, a preliminary image prior to the first live welding operation; analyze the preliminary image; and select image capture settings to be used for the capture of the images of the first live welding operation based on the analysis of the preliminary image. The preliminary image may be used as a point of reference for processing the images captured during the welding operation. For example, brightness, contrast, and/or other characteristics of the preliminary image (in which the arc is not present) may serve as baselines or targets to be achieved when processing the images captured during the live welding process (in which the arc is present and creating much more challenging lighting conditions).

The present methods and systems may be realized in hardware, software, or a combination of hardware and software. The present methods and/or systems may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may include a general-purpose computing system with a program or other code that, when being loaded and executed, controls the computing system such that it carries out the methods described herein. Another typical implementation may comprise an application specific integrated circuit or chip. Some implementations may comprise a non-transitory machine-readable (e.g., computer readable) medium (e.g., FLASH drive, optical disk, magnetic storage disk, or the like) having stored thereon one or more lines of code executable by a machine, thereby causing the machine to perform processes as described herein.

While the present system has been described with reference to certain implementations, it will be understood by those skilled in the art that various changes may be made and equivalents may be substituted without departing from the scope of the present system as defined in the appended claims. In addition, many modifications may be made to adapt a particular situation or material to the teachings of the present disclosure without departing from its scope. Therefore, it is intended that the present system not be limited to the particular implementations disclosed, but that the present system will include all implementations falling within the scope of the appended claims.

As utilized herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first set of one or more lines of code and may comprise a second "circuit" when executing a second set of one or more lines of code. As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g. and for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

## Claims

1. A system comprising:
- welding headwear (20) that comprises a camera (303), user interface components (308), a display (304), memory (411), and processing circuitry (410, 418), and that is operable to:
- capture, via the camera (303), images of a first live welding operation performed on a sample workpiece (24);
- store, to the memory (411), the captured images of the first live welding operation;
- play back, on the display (304), the stored images of the first live welding operation;
the system being **characterized in that** it is operable to
- display, on the display (304) multiple versions of one of the captured images of the first live welding operation, wherein each of the multiple versions of the one of the captured images of the first live welding operation is displayed with different image display settings,
- select, with or without user input, during the play back and based on the captured images, image display and/or capture settings of the welding headwear (20) to be used for a second live welding operation;
- capture, via the camera (303), images of a second live welding operation using the selected image capture settings, and
- display, on the display (304) in real-time, the images of the second live welding operation,
wherein the welding headwear (20) is operable to apply the selected images display and/or capture settings to the images of the second live weld operation during the display of the images of the second live weld operation.

2. The system of claim 1,
wherein the image display settings comprise one or more of: brightness, contrast, color, saturation, sharpness, saturation, and hue.

3. The system of claim 2,
wherein the camera (303) comprises one or more optical components (302) and image sensors (416), wherein
- image capture settings comprise settings of the optical components (302) of the camera (303); and
- the settings of the optical components (302) comprise one or more of: focal length, aperture, and exposure time.

4. The system of claim 1,
wherein the image display settings comprise settings for parameters of an algorithm for combining pixel data from a plurality of the image sensors (416).

5. The system of claim 4, wherein:
- the image capture settings comprise settings of an image sensor (416) of the camera (303); and
- the settings of the image sensor (416) comprise one or more of: exposure time, bias voltage, and bias current.

6. The system of one of the preceding claims,
wherein the welding headwear (20) is operable to configure the camera (303) to use, during the capture of the images of the first live weld operation, different image capture settings for different ones of the captured images of the first live welding operation.

7. The system of claim 6,
wherein the welding headwear (20) is operable to display, on the display (304), different ones of the captured images side-by-side during the play back.

8. The system of one of the preceding claims,
wherein the welding headwear (20) is operable to perform the selection automatically based on weld characteristics.

9. The system of claim 8,
wherein the welding headwear (20) is operable to determine the weld characteristics based on processing of the captured images of the first live welding operation.

10. The system of one of the preceding claims,
wherein the welding headwear (20) is operable to:
- capture, via the camera (303), a preliminary image prior to the first live welding operation;
- analyze the preliminary image; and
- select image capture settings to be used for the capture of the images of the first live welding operation based on the analysis of the preliminary image.

## Patentansprüche

1. System, aufweisend:
- Schweißkopfbedeckung (20), die eine Kamera (303), Benutzerschnittstellenkomponenten (308), eine Anzeige (304), einen Speicher (411) und eine Verarbeitungsschaltung (410, 418) aufweist, und die betreibbar ist zum:
- Erfassen, über die Kamera (303), von Bildern eines ersten Live-Schweißvorgangs, der an einem Musterwerkstück (24) durchgeführt wird;
- Speichern, in dem Speicher (411), der erfassten Bilder des ersten Live-Schweißvorgangs;
- Wiedergeben, auf der Anzeige (304), der gespeicherten Bilder des ersten Live-Schweißvorgangs;
das System **dadurch gekennzeichnet, dass** es betreibbar ist zum
- Anzeigen, auf der Anzeige (304), von mehreren Versionen eines der erfassten Bilder des ersten Live-Schweißvorgangs, wobei jede der mehreren Versionen des einen der erfassten Bilder des ersten Live-Schweißvorgangs mit unterschiedlichen Bildanzeigeeinstellungen angezeigt wird,
- Auswählen, mit oder ohne Benutzereingabe, während der Wiedergabe und basierend auf den erfassten Bildern, von Bildanzeige- und/oder Erfassungseinstellungen der für einen zweiten Live-Schweißvorgang zu verwendenden Schweißkopfbedeckung (20);
- Erfassen, über die Kamera (303), von Bildern eines zweiten Live-Schweißvorgangs unter Verwendung der ausgewählten Bilderfassungseinstellungen, und
- Anzeigen, auf der Anzeige (304) in Echtzeit, der Bilder des zweiten Live-Schweißvorgangs,
wobei die Schweißkopfbedeckung (20) zum Anwenden der ausgewählten Bildanzeige- und/oder Erfassungseinstellungen auf die Bilder des zweiten Live-Schweißvorgangs während der Anzeige der Bilder des zweiten Live-Schweißvorgangs betreibbar ist.

2. System nach Anspruch 1,
wobei die Bildanzeigeeinstellungen eine(s) oder mehrere aufweisen von: Helligkeit, Kontrast, Farbe, Sättigung, Schärfe, Sättigung und Farbton.

3. System nach Anspruch 2,
wobei die Kamera (303) eine oder mehrere optische Komponenten (302) und Bildsensoren (416) aufweist, wobei
- Bilderfassungseinstellungen Einstellungen der optischen Komponenten (302) der Kamera (303) aufweisen; und
- die Einstellungen der optischen Komponenten (302) eine oder mehrere aufweisen von: Brennweite, Blende und Belichtungszeit.

4. System nach Anspruch 1,
wobei die Bildanzeigeeinstellungen Einstellungen für Parameter eines Algorithmus zum Kombinieren von Pixeldaten von einer Vielzahl der Bildsensoren (416) aufweisen.

5. System nach Anspruch 4, wobei:
- die Bilderfassungseinstellungen Einstellungen eines Bildsensors (416) der Kamera (303) aufweisen; und
- die Einstellungen des Bildsensors (416) eine(s) oder mehrere aufweisen von: Belichtungszeit, Vorspannung und Vorstrom.

6. System nach einem der vorhergehenden Ansprüche,
wobei die Schweißkopfbedeckung (20) zum Konfigurieren der Kamera (303) zum Verwenden verschiedener Bilderfassungseinstellungen für verschiedene der erfassten Bilder des ersten Live-Schweißvorgangs während des Erfassens der Bilder des ersten Live-Schweißvorgangs betreibbar ist.

7. System nach Anspruch 6,
wobei die Schweißkopfbedeckung (20) zum Anzeigen, auf der Anzeige (304), verschiedener der erfassten Bilder nebeneinander während der Wiedergabe betreibbar ist.

8. System nach einem der vorhergehenden Ansprüche,
wobei die Schweißkopfbedeckung (20) zum automatischen Durchführen der Auswahl basierend auf den Schweißeigenschaften betreibbar ist.

9. System nach Anspruch 8,
wobei die Schweißkopfbedeckung (20) zum Ermitteln der Schweißeigenschaften basierend auf der Verarbeitung der erfassten Bilder des ersten Live-Schweißvorgangs betreibbar ist.

10. System nach einem der vorhergehenden Ansprüche,
wobei die Schweißkopfbedeckung (20) betreibbar ist zum:
- Erfassen, über die Kamera (303), eines vorläufigen Bildes vor dem ersten Live-Schweißvorgang;
- Analysieren des vorläufigen Bildes; und
- Auswählen von für das Erfassen der Bilder des ersten Live-Schweißvorgangs zu verwendenden Bilderfassungseinstellungen basierend auf der Analyse des vorläufigen Bildes.

## Revendications

1. Système comprenant :
- un casque de soudage (20) qui comprend une caméra (303), des éléments d'interface utilisateur (308), un affichage (304), une mémoire (411), et des circuits de traitement (410, 418), et qui est utilisable pour :
- la capture, via la caméra (303), d'images d'une première opération de soudage en direct effectuée sur une pièce à usiner échantillon (24) ;
- le stockage, dans la mémoire (411), des images capturées de la première opération de soudage en direct ;
- la lecture, sur l'affichage (304), des images stockées de la première opération de soudage en direct ;
le système étant **caractérisé en ce qu'**il est utilisable pour
- afficher, sur l'affichage (304) plusieurs versions de l'une parmi les images capturées de la première opération de soudage en direct, dans lesquelles chacune parmi les multiples versions de l'une parmi les images capturées de la première opération de soudage en direct est affichée avec différents paramètres d'affichage d'image,
- sélectionner, avec ou sans entrée utilisateur, durant la lecture et sur base des images capturées, Les paramètres d'affichage d'images et/ou de capture du casque de soudage (20) à utiliser pour une deuxième opération de soudage en direct ;
- la capture, via la caméra (303), d'images d'une deuxième opération de soudage en direct à l'aide des paramètres de capture d'image sélectionnés, et
- afficher, sur l'affichage (304) en temps réel, des
images de la deuxième opération de soudage en direct, dans lequel le casque de soudage (20) est utilisable pour appliquer les paramètres d'affichage d'images sélectionnées et/ou de capture aux images de la deuxième opération de soudage en direct durant l'affichage des images de la deuxième opération de soudage en direct.

2. Système selon la revendication 1,
dans lequel les paramètres d'affichage d'images comprennent un ou plusieurs parmi : luminosité, contraste, couleur, saturation, netteté, saturation et tonalité.

3. Système selon la revendication 2,
dans lequel la caméra (303) comprend un ou plusieurs éléments optiques (302) et des capteurs d'image (416), dans lequel
- les paramètres de capture d'image comprennent des paramètres des éléments optiques (302) de la caméra (303) ; et
- les paramètres des éléments optiques (302) comprennent un ou plusieurs parmi : longueur focale, ouverture et temps d'exposition.

4. Système selon la revendication 1,
dans lequel les paramètres d'affichage d'images comprennent des paramètres pour le paramétrage d'un algorithme pour la combinaison des données de pixels provenant d'une pluralité des capteurs d'image (416).

5. Système selon la revendication 4, dans lequel :
- les paramètres de capture d'image comprennent des paramètres d'un capteur d'image (416) de la caméra (303) ; et
- les paramètres du capteur d'image (416) comprennent un ou plusieurs parmi : temps d'exposition, tension de polarisation et courant de polarisation.

6. Système selon l'une quelconque des revendications précédentes,
dans lequel le casque de soudage (20) est utilisable pour configurer la caméra (303) pour utiliser, durant la capture des images de la première opération de soudage en direct, différents paramètres de capture d'image pour celles différentes parmi les images capturées de la première opération de soudage en direct.

7. Système selon la revendication 6,
dans lequel le casque de soudage (20) est utilisable pour afficher, sur l'affichage (304), celles différentes parmi les images capturées côte à côte durant la lecture.

8. Système selon l'une quelconque des revendications précédentes,
dans lequel le casque de soudage (20) est utilisable pour effectuer la sélection automatiquement sur base des caractéristiques de soudure.

9. Système selon la revendication 8,
dans lequel le casque de soudage (20) est utilisable pour déterminer les caractéristiques de soudure sur base du traitement des images capturées de la première opération de soudage en direct.

10. Système selon l'une quelconque des revendications précédentes,
dans lequel le casque de soudage (20) est utilisable pour :
- la capture, via la caméra (303), d'une image préliminaire avant la première opération de soudage en direct ;
- analyser l'image préliminaire ; et
- sélectionner des paramètres de capture d'image à utiliser pour la capture des images de la première opération de soudage en direct sur base de l'analyse de l'image préliminaire.
